# EUROPÄISCHE PATENTSCHRIFT

(11) **EP 3 968 902 B1**
(45) Veröffentlichungstag und Bekanntmachung des Hinweises auf die Patenterteilung: **04.11.2026**
(21) Anmeldenummer: 20734316.1
(22) Anmeldetag: 13.05.2020
(51) Int. Cl.: A61F 2/16

(54) **OPHTHALMOLOGISCHES IMPLANTAT, ERFAHREN ZU SEINER HERSTELLUNG, COMPUTERPROGRAMPRODUKT ZU SEINER HERSTELLUNG**
OPHTHALMIC IMPLANT, METHOD FOR ITS MANUFACTURE, COMPUTER PROGRAM PRODUCT FOR ITS MANUFACTURE
IMPLANT OPHTALMOLOGIQUE, PROCÉDÉ POUR SA FABRICATION, PRODUIT PROGRAMME D'ORDINATEUR POUR SA FABRICATION

(30) Priorität: 16.05.2019 DE 102019207175
(43) Veröffentlichungstag der Anmeldung: 23.03.2022
(73) Patentinhaber: Carl Zeiss Meditec AG, 07745 Jena (DE)
(72) Erfinder: GERLACH, Mario, 10589 Berlin (DE)
(74) Vertreter: Rößner, Ulrike
(86) Internationale Anmeldenummer: PCT/EP2020/063332
(87) Internationale Veröffentlichungsnummer: WO 2020/229540

(56) Entgegenhaltungen:
- EP-A2- 1 081 640
- DE-A1- 102009 056 810
- US-A1- 2006 001 828
- US-B2- 8 812 125

## Beschreibung

Die vorliegende Erfindung betrifft ein ophthalmologisches Implantat, das ein optisch abbildendes Element, insbesondere eine zentrale optische Linse, und eine Haptik mit einer Haptikwurzel, die sich dem optisch abbildenden Element anschließt umfasst. Ein solches ophthalmologisches Element ist insbesondere eine Intraokularlinse. Die vorliegende Erfindung betrifft weiterhin ein entsprechendes Verfahren zur Herstellung eines ophthalmologischen Implantats, in dem das ophthalmologische Implantat mittels eines spanabhebenden Drehverfahrens, eines Laserstrahlformverfahrens oder eines Urformverfahrens geformt wird. Zudem betrifft die Erfindung ein Charakterisierungssystem zur Identifikation eines ophthalmologischen Implantats, insbesondere einer Intraokularlinse, ein Computerprogrammprodukt und ein computerlesbares Medium.

Die meisten ophthalmologischen Implantate, insbesondere die aus künstlichen Materialien hergestellten, bei denen es sich mehrheitlich um kommerzielle Intraokularlinsen (IOL) handelt, werden mittels Label auf der Primär- und Sekundärverpackung gekennzeichnet. Auf dem Label findet man neben Herstellerinformationen, beispielsweise den Typ der Intraokularlinse sowie ihren Brechwert. Die korrekte Versorgung des Patienten setzt somit voraus, dass die verpackte und ausgelieferte Intraokularlinse bzw. anderes ophthalmologisches Implantat in ihren Eigenschaften den Angaben auf den Labeln der Verpackungen entspricht. Der Anwender muss sich hierbei auf die Hersteller verlassen, da eine eindeutige Identifikation bzw. eine Überprüfung im Operationssaal nur anhand der visuellen Linsenmerkmale schwierig ist. Es besteht deshalb die Gefahr, dass es auf Seiten der Hersteller zu Verwechslungen und fehlerhafter Kennzeichnung kommt, was in der Konsequenz unter Umständen Explantationen nach sich ziehen würde, um dem Patienten dann beispielsweise eine neue, korrekte Intraokularlinse zu implantieren. Somit würde der Patienten mehrfach belasten. Zudem führt die bloße Vermutung, dass eine Verwechslung stattgefunden haben könnte, zu kostspieligen und rufschädigenden Produktrückrufen.

Deshalb wurden beispielsweise in der WO 2009/124838 A2 Verfahren vorgeschlagen, die Intraokularlinsen mit IR-Kennzeichnungen zu markieren, die mittels Anwendung fluoreszierender Farbstoffe realisiert werden. Eine weitere bekannte Möglichkeit der individuellen Kennzeichnung ist die Anwendung lasergravierter Matrixcodes in der Haptik nach DE 10 2009 056 810 A1, die in einem zusätzlichen Verfahren nach der Erzeugung des ophthalmologischen Implantats erzeugt wird. Diese Verfahren sind jedoch technisch sehr aufwändig und erfordern beispielsweise im Falle der IR-Kennzeichnungen einen zusätzlichen biokompatiblen Fluoreszenzfarbstoff und ein komplexes Fluoreszenzanregungs- und Detektionssystem. Die Markierung der Haptik mittels lasergraviertem Matrixcode setzt hingegen ein entsprechendes Lasersystem und den Nachweis der Biokompatibilität des Verfahrens voraus. Alle bislang bekannten Verfahren müssen derart in die Fertigungskette eingebunden werden, dass keine falsche Kennzeichnung der Intraokularlinse durch Bedien- oder Programmierfehler möglich ist. Da es sich hier jeweils im zusätzliche Herstellungsschritte in separaten Verfahren und mit separaten Vorrichtungen handelt, bleibt trotzdem ein Restrisiko der Verwechslung bestehen.

Zudem bleibt immer ein Risiko der Beeinträchtigung des optisch abbildenden Elements, sobald die Markierung auf dem optisch abbildenden Element aufgebracht werden soll, damit sie einfacher auffindbar und lesbar ist. Solche Markierungen müssen sehr platzsparend erfolgen. Die US 2006/0001828 A1 beschreibt Kontaktlinsen mit Markierungen auf dem optisch abbildenden Element, die so konzipiert werden, dass eine zweite Markierung innerhalb einer ersten Markierung erfolgt.

Aufgabe der vorliegenden Erfindung ist es deshalb, eine eindeutige Kennzeichnung mindestens des Typs und der Brechkraft auf einem ophthalmologischen Implantat, insbesondere auf einer Intraokularlinse, und damit eine eindeutige und sichere Identifikationsmöglichkeit zu realisieren, ohne dass zusätzliche Additive, Biomaterialien, Chemikalien oder neue Herstellungsverfahren benötigt werden. Die Aufbringung der Kennzeichnung soll zwangsgeführt, verwechslungssicher und mit minimalem technischen Zusatzaufwand möglich sein.

Die Erfindung ist in den unabhängigen Ansprüchen definiert. Die abhängigen Ansprüche betreffen bevorzugte Weiterbildungen.

Ein ophthalmologisches Implantat umfasst ein optisch abbildendes Element mit einer Vorderseite und einer Rückseite sowie einem umlaufenden Rand. An diesem umlaufenden Rand des optisch abbildenden Elements (oder zumindest in dessen unmittelbarer Nähe) kann und muss dieses abbildende optische Element gehalten werden, da alle anderen Haltepositionen die optische Nutzbarkeit des Elements einschränken würden. Zum Halten, Stabilisieren, Positionieren bzw. Zentrieren und Fixieren am Anwendungsort in einem Patientenauge umfassen ophthalmologische Implantate deshalb des Weiteren eine Haptik mit einer Haptikwurzel, die sich dem optisch abbildenden Element anschließt, insbesondere dieses an seinem umlaufenden Rand mindestens teilweise umschließt und somit einen sicheren Halt gewährt. Es besteht also in der Regel eine feste Verbindung des optisch abbildenden Elements mit der Haptik über die Haptikwurzel. Unter den Begriff Haptik soll dabei jeglicher peripher zur Optik befindlicher Körper eines solchen ophthalmologischen Implantats fallen.

Das optisch abbildende Element des ophthalmologischen Implantats ist in der Regel eine zentrale optische Linse. Diese wird von einer sich zumeist an mehreren Positionen des umlaufenden Rands dieser Linse anschließenden Haptik gehalten.

Die größte Gruppe der ophthalmologischen Implantate betrifft, wie oben schon erwähnt, Intraokularlinsen. Diese (wie auch die meisten anderen ophthalmologischen Implantate) werden aus einem biokompatiblen künstlichen Material, in der Regel einem Polymer oder einem anderen organischen Material, ggf. aber auch aus einem Glas, insbesondere einem Silikatglas, hergestellt. Ophthalmologische Implantate aus der speziellen Gruppe der Transplantate (und üblicherweise auch so bezeichnet) sollen nicht unter die Erfindung fallen.

Erfindungsgemäß ist nun auf der Haptikwurzel und/oder dem der Haptikwurzel nahen Bereich der Haptik eine strukturelle Codierung von Identifikationsdaten des ophthalmologischen Implantats, insbesondere des Typs und der Brechkraft, angeordnet.

Als "strukturelle Codierung" wird hier eine Codierung mit einer Struktur, die eine Topographie aufweist, verstanden, also eine in ihrer Form, in ihrer Morphologie, erkennbare Codierung. Eine solche "strukturelle Codierung" ist sehr vorteilhaft im selben Formungsverfahren wie die das ophthalmologische Implantat erzeugt, also im Prinzip als eine "in-situ strukturelle Codierung".

Die Identifikationsdaten des ophthalmologischen Implantats umfassen die charakterisierenden Parameter dieses ophthalmologischen Implantats, stellen also die Kennzeichnung des ophthalmologischen Implantats dar, mit der dieses eindeutig identifiziert bzw. verifiziert werden kann. Wie oben erwähnt, betrifft das den Typ des ophthalmologischen Implantats, insbesondere einer Intraokularlinse, wie auch alle notwendigen Informationen zu optischen Eigenschaften, wie - im Falle der Intraokularlinse - deren Brechkraft.

Die Anordnung auf der Haptikwurzel bzw. auf der Haptik nahe der Haptikwurzel erlaubt eine sichere Identifizierung des ophthalmologischen Implantats, insbesondere einer Intraokularlinse, in verschiedenen Situationen: Sie ist leicht erkennbar und identifizierbar auf dem Implantat vor der Implantation, aber auch noch erreichbar zur Identifizierung beispielsweise einer Intraokularlinse nach der Implantation in das Patientenauge, wenn dieses hierfür "weitgetropft" wird.

In einer bevorzugten Ausgestaltung des erfindungsgemäßen ophthalmologischen Implantats ist die strukturelle Codierung der Identifikationsdaten des ophthalmologischen Implantats, also seine Kennzeichnung, mittels eines Barcodesystems realisiert. Ein Barcodesystem erlaubt eine binäre Codierung mittels sehr einfacher Strukturen. Je nach konkreter Realisierung des Barcodesystems erlaubt es eine größere Zahl von Daten zu hinterlegen und wieder auszulesen, so dass ggf. auch - neben dem Typ des ophthalmologischen Implantats und seiner optischen Daten - weitere Daten wie Herstellungsort, Herstellungsprozess, Datum etc., im Sinne einer eindeutig nachvollziehbaren Produktkennzeichnung hinterlegt werden können.

Um nun die allgemeine Idee der Erfindung weiter zu erläutern: Ophthalmologische Implantate, so auch Intraokularlinsen, weisen eine gewisse Rotationssymmetrie auf. Das optisch abbildende Element, in der Regel eine optische Linse, die möglichst zentral angeordnet ist, ist dann zumindest auf Teilbereichen ihres umlaufenden Randes von einer Haptik mit einer Haptikwurzel umgeben, in der die strukturelle (Im Sinne von topographischer) Codierung rotationssymmetrisch auf einer Haptikwurzel und/oder dem der Haptikwurzel nahem Bereich der Haptik, die das optisch abbildende Element an dessen umlaufenden Rand teilweise umschließt, angeordnet ist. Die Rotationssymmetrie wird dabei unterbrochen an Stellen, an denen sich an den umlaufenden Rand der Linse keine Haptik anschließt.

Im Prinzip handelt es sich hier also, sofern die Codierung als Barcode ausgestaltet ist, um einen ringförmigen Barcode, der im gesamten Bereich der Haptikwurzel und der der Haptikwurzel nahen Bereich der Haptik um den umlaufenden Rand des optisch abbildenden Elements herum realisiert ist. Die Ringform wird ggf. dort unterbrochen, wo das optisch abbildende Element, insbesondere die Intraokularlinse, nicht von einer Haptik umschlossen wird. Dabei sind vorzugsweise die wichtigsten Informationen - wie die Typenbezeichnung des ophthalmologischen Implantats und die entscheidenden optischen Eigenschaften, wie beispielsweise die Brechkraft einer entsprechenden Intraokularlinse, am nächsten zum optisch abbildenden Element angeordnet.

Im Falle einer solchen rotationssymmetrischen Ausführung der strukturellen Codierung durch einem rotationssymmetrischen (dort, wo keine Haptik vorhanden, unterbrochenen) kreisförmigen Barcode, ist das erfindungsgemäße ophthalmologische Implantat besonders einfach und sicher realisierbar: Sowohl die Formung des ophthalmologischen Implantats als auch die strukturelle Codierung kann beispielsweise mit einem und demselben spanabhebenden Drehverfahren, insbesondere einem Diamantdrehverfahren, aber auch mit einem Laserstrahlformungsverfahren (in dem entweder das zu formende ophthalmologische Implantat gedreht wird, während der Laserstrahl entlang eines Radius der Linse über die Linse geführt wird, oder ein Rotationsscan inklusive einer Verschiebung des Laserstrahls entlang eines Radius der Linse mit unbeweglichem zu formenden ophthalmologischen Implantat realisiert wird), ausgeführt werden. Der so erzeugte rotationssymmetrische Barcode erlaubt eine strukturelle Codierung des ophthalmologischen Implantats, die so angeordnet ist, dass sie in verschiedenen Situationen, insbesondere auch nach der Implantation, leicht auslesbar ist.

Die strukturelle Codierung des erfindungsgemäßen ophthalmologischen Implantats ist bevorzugt durch ein Set räumlicher Modulationsparameter entsprechender zur Codierung genutzter Strukturen auf der Haptikwurzel und der Haptik bestimmt.

Eine bevorzugtes erfindungsgemäßes ophthalmologisches Implantat ist gekennzeichnet durch eine Codierung, die durch mindestens einen der folgenden Modulationsparameter beschrieben ist: Furchenbreite, Furchentiefe, Neigungswinkel der Furche, Position der Furche, insbesondere eine radiale Position der Furche, beschrieben durch den Radius der Furchenmitte der jeweiligen Furche für eine rotationssymmetrische Codierung. Als Furche sei hier eine Vertiefung, Einkerbung oder ein mit verschiedenen Mitteln geschaffener "Graben" im Material der Haptikwurzel und der haptikwurzelnahen Haptik des ophthalmologischen Implantats bezeichnet. Bei einer nichtsenkrecht eingebrachten Furche bezeichnet die Furchentiefe die Maximaltiefe dieser Furche, die Furchenbreite die Breite der Furche an der Oberkante, und eine Krümmung einer Furchenwand kann ein zu einem Neigungswinkel einer schrägen, aber ebenen Furchenwand gleichwertiger Modulationsparameter sein.

Um in der Codierung die gewünschten Daten zu hinterlegen, muss also mindestens ein entsprechender räumlicher Modulationsparameter variiert werden. Wieviel und welche Modulationsparameter für die strukturelle Codierung genutzt werden können, hängt auch davon ab, mit welchem optischen Detektionsverfahren der räumliche Modulationsparameter am besten erfassbar ist, und ob und wie ein solches Detektionsverfahren bzw. ein entsprechendes Charakterisierungssystem zur Verfügung steht.

Je nach Ausgestaltung ist also für die Codierung mindestens ein binäres Arbeiten möglich. Zur räumlichen Kompression der Datenstruktur sind jedoch auch höhere Auflösungen möglich, wenn beispielweise nicht einfach nur eine Feststellung getroffen werden kann, dass eine Furche an einer definierten Position, beispielsweise mit einem definiertem Radius zu einem Mittelpunkt des optisch abbildenden Elements, vorhanden ist, sondern verschiedene Furchentiefen detektierbar sind.

Kann eine derartige Kompression der Datenstruktur genutzt werden, so können je nach dem erreichbaren räumlichen Auflösungsvermögen der Datenstruktur weitere Kennzeichen, Redundanzbits, Prüfsummen oder Start-Stopp Kennzeichnungen eingebracht werden.

Von besonderem Vorteil ist es, wenn idealerweise das gesamte erfindungsgemäße ophthalmologische Implantat aus einem Werkstück gefertigt worden ist, zumindest aber das optisch abbildende Element und die Haptik mit ihrer Haptikwurzel aus einem Werkstück gefertigt sind. In der Regel wird ein solches ophthalmologisches Implantat beispielsweise aus einem biokompatiblen Polymermaterial herausgearbeitet, bevorzugt durch ein Diamantdrehverfahren. Möglich sind für die Herausarbeitung aber auch, wie bereits erwähnt, jegliches spanabhebendes Drehverfahren oder ein Laserstrahlformungsverfahren. Alternativ bevorzugt kann das ophthalmologische Implantat aus einem biokompatiblen Polymermaterial auch durch Urformverfahren (Formgießen, Spritzgießen) hergestellt werden.

Dabei ist es sehr vorteilhaft, wenn in einem erfindungsgemäßen ophthalmologischen Implantat die strukturelle Codierung während oder direkt nach der Formung des ophthalmologischen Implantats ausgebildet ist. Formung und Codierung erfolgen dann in einem Arbeitsschritt, ohne dass das sich in Arbeit befindliche ophthalmologische Implantat einen Ortswechsel vollziehen muss. Fertigungsdaten der Formung des ophthalmologischen Implantats können dann direkt zur Codierung genutzt werden. In diesem Fall wird die höchstmögliche Sicherheit für eine spätere fehlerfreie Identifikation des ophthalmologischen Implantats erreicht.

In einem erfindungsgemäßen Verfahren zur Herstellung eines ophthalmologischen Implantats, insbesondere einer Intraokularlinse, wird das ophthalmologische Implantat mittels eines spanabhebenden Drehverfahrens, insbesondere mittels eines Diamantdrehverfahrens geformt, und es erfolgt direkt während oder nach der Formung des ophthalmologischen Implantats eine strukturelle Codierung von Identifikationsdaten des ophthalmologischen Implantats, insbesondere des Typs und der Brechkraft, mittels des spanabhebenden Drehverfahrens (insbesondere mittels des Diamantdrehverfahrens wenn ein solches eingesetzt wird) - zur einfachen, sicheren und eindeutigen Kennzeichnung des Implantats. Hierzu müssen keine Maschinen gewechselt werden, das ophthalmologische Implantat verbleibt auf seinem Platz. Das Diamantdrehverfahren ist ein hierfür besonders geeignetes Verfahren zur Herstellung eines ophthalmologischen Implantats, insbesondere einer Intraokularlinse. Jedoch können auch andere spanabhebende Drehverfahren eingesetzt werden, wie beispielsweise das Präzisionsdrehverfahren.

Alternativ wird in einem erfindungsgemäßen Verfahren zur Herstellung eines ophthalmologischen Implantats, insbesondere einer Intraokularlinse, das ophthalmologische Implantat mittels eines Laserstrahlformungsverfahrens geformt. Auch hier erfolgt die strukturelle Codierung von Identifikationsdaten des ophthalmologischen Implantats während oder nach der Formung des ophthalmologischen Implantats mit demselben Verfahren, also in diesem Falle mit dem Laserstrahlformungsverfahren. In einem solchen Laserstrahlformungsverfahren wird entweder das zu formende ophthalmologische Implantat gedreht, während der Laserstrahl entlang eines Radius der Linse über die Linse geführt wird, oder es wird ein Rotationsscan des Laserstrahls inklusive einer Verschiebung des Laserstrahls entlang eines Radius der Linse mit unbeweglichem zu formenden ophthalmologischen Implantat realisiert.

Diese strukturelle Codierung durch das Laserstrahlformverfahren umfasst auch in diesem Fall insbesondere den Typ und der Brechkraft zur einfachen, sicheren und eindeutigen Kennzeichnung des Implantats. Hierzu müssen auch hier keine Maschinen gewechselt werden, das ophthalmologische Implantat verbleibt auf seinem Platz.

Ein solches Laserstrahlformungsverfahren kann mit einem Laser arbeiten, der mittels Ablation Material abträgt wie beispielsweise einem Excimer-Laser oder einem IR-Laser. Es kann aber auch mit einem Laser arbeiten, der mittels Photodisruption das Material "schneidet" bzw. an der Stelle der Fokuswirkung auf bzw. im Material dieses entfernt, wie beispielsweise einem Femtosekundenlaser oder einem Picosekundenlaser (also ganz allgemein einem gepulsten Laser). In beiden Fällen benötigt ein entsprechendes Lasersystem, mit dem eine solches Laserstrahlformungsverfahren durchgeführt werden soll, einen Scanner, der den Fokus des Laserstrahls auf bzw. im Material in allen drei Raumrichtungen (bzw. im Falle eines Ablationslasers mindestens in den beiden Raumrichtungen senkrecht der optischen Achse bzw. der Ausbreitungsrichtung des Laserstrahls) entsprechend eines für die gewünschte Formung vorgegebenen Musters bewegt.

Die strukturelle Codierung von Identifikationsdaten des ophthalmologischen Implantats erfolgt dabei in der Regel dicht neben dem optisch abbildenden Element des ophthalmologischen Implantats auf seiner Haptik, insbesondere auf seiner Haptikwurzel, so dann die optische Funktion des ophthalmologischen Implantats nicht gestört wird, die Identifikation des ophthalmologischen Implantats jedoch in vielen Positionen, ggf. auch noch nach seiner Implantation in ein Patientenauge möglich ist.

Die strukturelle Codierung von Identifikationsdaten des ophthalmologischen Implantats erfolgt also erfindungsgemäß mit demselben Verfahren wie die Formung des ophthalmologischen Implantats - im Anschluss an die Formung oder sogar schon während der Formung des ophthalmologischen Implantats. Es erfolgt kein Ortswechsel des ophthalmologischen Implantats, was die Gefahr des Vertauschens bzw. Verwechselns von ophthalmologischen Implantaten, wie sie beispielsweise bei einer Manipulation des ophthalmologischen Implantats zwischen einem Formungsverfahren und einem separaten Codierungsverfahren bestehen würde, ausschließt.

Für die Intraokularlinsen als wichtigste Gruppe der ophthalmologischen Implantate wird die Aufgabe beispielsweise bevorzugt dahingehend gelöst, dass mittels eines für ihre Herstellung bestehenden Diamantdrehverfahren oder aber anderweitigen spanabhebenden Drehverfahrens eine rotationsymmetrische strukturelle Codierung in einem Bereich der Haptikwurzel der Intraokularlinse, und damit in nächster Nähe zur zentralen optischen Linse, eingebracht wird.

In einer besonders vorteilhaften Variante des erfindungsgemäßen Verfahrens werden Daten, insbesondere Steuerdaten (wie z.B. für das Diamantdrehverfahren oder einem anderen spanabhebenden Drehverfahren oder einem Laserstrahlformungsverfahren), zur Durchführung der strukturellen Codierung von Identifikationsdaten des ophthalmologischen Implantats aus Steuerdaten zur Formung des ophthalmologischen Implantats und/oder aus Kontrolldaten der Formung des ophthalmologischen Implantats gewonnen.

Die Steuerdaten zur Formung des ophthalmologischen Implantats sind dabei die Daten zur direkten Steuerung einer entsprechenden Drehmaschine, wie die Daten aus einer CNC-Datei. Kontrolldaten sind Daten aus Rückmeldungen der Drehmaschine, wie beispielsweise die Rückmeldung der Lage des Diamants oder eines anderen spanabhebenden Werkzeugs (in Abhängigkeit von Ort und Zeit) beim Schleifen.

Die Information zur Maschinensteuerung zum sogenannten Konturschnitt (also der Formung des ophthalmologischen Implantats) sind von der Datenstruktur her in der jeweiligen CNC-Datei des ophthalmologischen Implantats, hier vorzugsweise der Intraokularlinse des entsprechenden Brechwerts und Typs, fest angelegt. Somit enthält bei Anwendung des erfindungsgemäßen Verfahrens, jedes mittels einer der vorher erstellten und gekennzeichneten CNC-Dateien hergestellte ophthalmologische Implantat, insbesondere jede entsprechende Intraokularlinse zwangsläufig und direkt die hierzu passende topographische Kennzeichenstruktur, also die entsprechende strukturelle Codierung (z.B. Typ ABCD @ 20.0D). Eine Verwechslung ist durch diese Zwangskopplung ausgeschlossen. Mit dieser Kennzeichnung ist das ophthalmologische Implantat, also insbesondere die Intraokularlinse ab der modellprägenden Formgebung auf der Drehmaschine eindeutig, für eine IOL mindestens in Typ und Brechwert, gekennzeichnet. Damit können an sämtlichen notwendigen Stellen im Herstellprozess, Verpackungsprozess, Auslieferungsprozess, vor und während einer Operation, und ggf. auch nach einer Implantation in ein Patientenauge die das ophthalmologische Implantat kennzeichnenden Daten, wie Typ und Brechwert einer Intraokularlinse, eindeutig ermittelt und mit der Dokumentation oder Kennzeichnungsmitteln abgeglichen werden.

Alternativ sind die Steuerdaten zur Formung des ophthalmologischen Implantats - bei Nutzung eines Laserstrahlformungsverfahrens - Daten zur räumlichen Steuerung des Laserfokus (also Steuerdaten des Scanners eines Lasersystems) und Daten zur Steuerung der Leistung und ggf. der Pulsdauer des Lasers. Kontrolldaten können hier beispielsweise Rückmeldedaten des Scanners bzw. des Lasers oder aber Daten von einer im Lasersystem integrierten zusätzlichen Charakterisierungsvorrichtung, mit der die Formung des ophthalmologischen Implantats mittels des Lasersystem direkt charakterisiert werden kann, sein.

In einem weiteren alternativen erfindungsgemäßen Verfahren zur Herstellung eines ophthalmologischen Implantats, insbesondere einer Intraokularlinse, wird das ophthalmologische Implantat mittels eines Urformverfahrens geformt, in dem eine formgebende Übertragung der gewünschten Form des ophthalmologischen Implantats von einer Urform erfolgt, und es erfolgt direkt während der Formung des ophthalmologischen Implantats eine strukturelle und rotationssymmetrische Codierung von Identifikationsdaten des ophthalmologischen Implantats, insbesondere des Typs und der Brechkraft, durch die Urform - zur einfachen, sicheren und eindeutigen Kennzeichnung des Implantats. Hierzu müssen wiederum keine Maschinen gewechselt werden, Formung und Codierung erfolgen im selben Verfahren. In diesem Falle enthält die Urform bereits die notwendige strukturelle und rotationssymmetrische Codierung von Identifikationsdaten des ophthalmologischen Implantats, insbesondere des Typs und der Brechkraft zur formgebenden Übertragung auf das Implantat als Negativ der dann auf dem ophthalmologischen Implantat vorgesehenen strukturellen Codierung. Ein besonders bevorzugtes Urformverfahren hierfür ist das Spritzgussverfahren. Es kann aber auch das Verfahren der Polymerisation in der Urform oder eine anderes Formgussverfahren eingesetzt werden.

Die strukturelle Codierung von Identifikationsdaten des ophthalmologischen Implantats erfolgt dabei auch hier vorzugsweise dicht neben dem optisch abbildenden Element des ophthalmologischen Implantats auf seiner Haptik, insbesondere auf seiner Haptikwurzel, so dann die optische Funktion des ophthalmologischen Implantats nicht gestört wird, die Identifikation des ophthalmologischen Implantats jedoch in vielen Positionen, ggf. auch noch nach seiner Implantation in ein Patientenauge möglich ist, wofür sich eine rotationssymmetrische strukturelle Codierung besonders eignet.

Für die Intraokularlinsen als wichtigste Gruppe der ophthalmologischen Implantate wird die Aufgabe bevorzugt dahingehend gelöst, dass mittels des für ihre Herstellung bestehenden Urformverfahrens eine rotationsymmetrische strukturelle Codierung in einem Bereich der Haptikwurzel der Intraokularlinse, und damit in nächster Nähe zur zentralen optischen Linse, eingebracht wird.

Wird ein Urformverfahren zur Herstellung eines ophthalmologischen Implantats eingesetzt, ohne dass eine Nachbearbeitung des ophthalmologischen Implantats erfolgt, dann ist es weiterhin von besonderem Vorteil, wenn die Daten zur Durchführung der strukturellen Codierung von Identifikationsdaten des ophthalmologischen Implantats aus Steuerdaten zur Erstellung einer Urform zur Formung des ophthalmologischen Implantats und/oder aus Kontrolldaten der Erstellung der Urform zur Formung des ophthalmologischen Implantats gewonnen und während oder direkt nach der Erstellung der Urform, beispielsweise mittels eines Drehverfahrens oder eines Laserstrahlformungsverfahrens, im selben Verfahren in der Urform (als Negativ) abgelegt werden.

Eine weitere Möglichkeit der Herstellung eines ophthalmologischen Implantats besteht in einer Kombination aus einem Urformverfahren, insbesondere ein Spritzgussverfahren, durch das eine allgemeine Form des ophthalmologischen Implantats unter Nutzung einer allgemeinen Urform erzeugt wird. Im Anschluss kann dann ein Drehverfahren oder ein Laserstrahlformungsverfahren wie oben beschrieben in sehr effektiver Art und Weise genutzt werden, um dem ophthalmologischen Implantat die gewünschten speziellen (optischen) Eigenschaften in entsprechender Feinformgebung zu übertragen, und direkt während dieses Verfahrensschrittes auch eine diese Eigenschaften beschreibende rotationssymmetrische strukturelle Codierung durchzuführen.

Ein erfindungsgemäßes Charakterisierungssystem zur Identifikation eines oben beschriebenen ophthalmologischen Implantats, insbesondere einer Intraokularlinse, weist ein Beleuchtungssystem zur Beleuchtung einer strukturellen Codierung, ein Kamerasystem zur Aufnahme von mittels der Beleuchtung detektierbar gemachten Strukturen der strukturellen Codierung und eine Analyseeinheit zur Auswertung eines vom Kamerasystem aufgenommenen Bildes der mittels der Beleuchtung detektierbar gemachten Strukturen der strukturellen Codierung sowie zur Decodierung von Identifikationsdaten zur Identifikation des ophthalmologischen Implantats aus diesem Bild auf.

Als Beleuchtungssystem und als Kamerasystem bedarf dabei keiner besonderen Ausführungen, eine große Zahl üblicher Beleuchtungssysteme und Kamerasysteme sind hierfür nutzbar. Jedoch sollten Kamerasystem und Beleuchtungssystem so aufeinander abgestimmt sein, dass das Licht des Beleuchtungssystems durch das Kamerasystem ohne Weiteres detektierbar ist. Es bedarf dann eigentlich nur einer zusätzlichen Analyseeinheit, mit der eine Bildanalyse eines mit dem Kamerasystem aufgenommenen Bildes ermöglicht wird und die eingerichtet ist, dieses Bild zu Decodieren und somit in frei lesbare Angaben zu den Eigenschaften des ophthalmologischen Implantats "zu übersetzen".

Ein vorteilhaftes erfindungsgemäßes Charakterisierungssystem weist weiterhin eine Anzeige- und/oder Ausgabevorrichtung zur Anzeige und/oder Ausgabe der decodierten Identifikationsdaten des ophthalmologischen Implantats auf. Über eine Anzeigevorrichtung können die decodierten Identifikationsdaten des ophthalmologischen Implantats dem Nutzer direkt angezeigt werden. Eine Ausgabevorrichtung erlaubt vorzugsweise die Kommunikation auch mit anderen Systemen, kann also die Identifikationsdaten an andere Systeme ausgeben bzw. übermitteln. Dies kann über drahtgebundene oder nichtdrahtgebundene Kommunikationswege erfolgen. Auch die Möglichkeit zur Ablage auf einem Speichermedium zur Wiederaufnahme in anderen Systemen ist hier beinhaltet.

Besonders bevorzugt ist ein Charakterisierungssystem, in dem die Anzeige- und/oder Ausgabevorrichtung, vorzugsweise in Zusammenarbeit mit der Analyseeinheit, eingerichtet ist,
- die Identifikationsdaten des ophthalmologischen Implantats weiterzuverarbeiten, und/oder
- die Identifikationsdaten in einer externen Datenbank zu hinterlegen und/oder
- die Identifikationsdaten mit Daten einer externen Datenbank abzugleichen und/oder
- Rückmeldungen (wie beispielsweise Alarme, Warnungen oder Bestätigungen) an einen Benutzer zu geben.

Eine solche Datenbank kann beispielsweise ein Praxismanagementsystem, eine elektronische Patientenakte oder eine allgemeine Implantat-Datenbank sein. Zu letzterem zählt auch ein zentrales externes Implantat-Datenbank-System zur Registrierung und langfristigen oder gar zeitlich unbegrenzten Nachverfolgung von Implantaten.

Bei einem Abgleich der Identifikationsdaten mit einer externen Datenbank kann so beispielsweise die Übereinstimmung des identifizierten ophthalmologischen Implantats mit einem Eintrag in der Patientenakte abgeglichen werden. Bei einer Nichtübereinstimmung können dann an dieser Stelle sofort geeignete Gegenmaßnahmen (bzw. Alarme) getroffen werden, so dass eine Implantation eines falschen ophthalmologischen Implantats in ein Patientenauge an verschiedenen Stellen bis hin zum Operation selbst verhindert werden kann.

In einer Variante des erfindungsgemäßen Charakterisierungssystems ist das Beleuchtungssystem als Spaltbeleuchtungssystem ausgebildet, das eine einfache Art der Beleuchtung und Detektierbarmachung einer solchen strukturellen Codierung darstellt.

Besonders vorteilhaft ist ein erfindungsgemäßes Charakterisierungssystem in dem Beleuchtungssystem und Kamerasystem ausgebildet sind, mit Licht im nicht-sichtbaren Spektralbereich zu arbeiten. Zum Beispiel können mit einem Beleuchtungssystem und einem Kamerasystem, das im Nah-Infrarotbereich arbeitet, aber auch mit Licht anderer nicht-sichtbaren Wellenlängen Blendungen des Patientenauges vermieden werden. Dabei ist es nicht notwendig, die gesamte rotationssymmetrische strukturelle Codierung gleichzeitig zu beleuchten und auszulesen: Vielmehr reicht es, einen kleinen Bereich dieser Codierung so zu beleuchten, dass entlang eines Radius der Codierung dieser vollständig beleuchtet werden kann und damit sämtliche Informationen, die sich ja in diesem Radiusbereich an jeder Stelle der Haptik (und insbesondere der Haptikwurzel) befinden, zu erfassen. Die Situation ist damit sehr komfortabel, da nicht an einer ganz präzisen Stelle, die beispielsweise nach der Implantation riskiert, nicht mehr zugänglich zu sein, nach der Information gesucht werden muss, sondern einfach ein Bereich gewählt werden kann, in dem die Zugänglichkeit gegeben ist.

Ein erfindungsgemäßes Mikroskopiesystem insbesondere ein Operations-Mikroskopiesystem, enthält ein oben beschriebenes Charakterisierungssystem, sodass das System wie üblich zur Kontrolle bzw. Nachverfolgung der Implantation eines oben beschriebenen ophthalmologischen Implantats genutzt werden kann und zusätzlich die Identifikationsdaten des ophthalmologischen Implantats dekodiert werden können. Vorzugsweise können außerdem in einem solchen Mikroskopiesystem die decodierten Identifikationsdaten des ophthalmologischen Implantats in eine Mikroskopbildebene bzw. in den Beobachtungstrahlengang des Mikroskops eingeblendet werden. Der Nutzer hat damit neben den üblichen Informationen durch die Beobachtung des Operationsverlaufs stets Kenntnis der Identifikationsdaten des gerade zu implantierenden ophthalmologischen Implantats:
Das Auslesen der Datenstruktur, beispielsweise an einem Operationsmikroskop, ist damit stets möglich. So kann der Nutzer (i.d.R. der Chirurg) im Zweifelsfall den Typ und die optischen Daten des ophthalmologischen Implantats, beispielsweise Typ und Brechwert einer Intraokularlinse, während der Operation oder im Reklamationsfall jederzeit überprüfen.

Weiterhin ist es von Vorteil, wenn ein erfindungsgemäßes Mikroskopiesystem des Weiteren eingerichtet ist, das vom Kamerasystem aufgenommene Bild zur Erkennung von Lage- und Positionsänderungen des ophthalmologischen Implantats während einer Implantation in ein Patientenauge auszuwerten: Die strukturelle Codierung eignet sich sehr gut zur Nachverfolgung einer ggf. auch ungewollten Positionsänderung des Implantats, und hat damit - neben der Identifikation des ophthalmologischen Implantats einen zusätzlichen Nutzen bzw. liefert einen zusätzlichen Sicherheitsaspekt.

Idealerweise erfolgt die Auswertung der Datenstruktur der strukturellen Codierung und die Darstellung der Klardaten durch ein Charakterisierungssystem mit digitalem Kamerasystem. Dieses kann, wie hier beschrieben, mit einem visuellen Beobachtungsmikroskop gekoppelt oder aber in einem speziellen Prüfmittel angeordnet sein.

Ein erfindungsgemäßes Computerprogrammprodukt enthält Programm-Code, der bei seiner Ausführung auf einem Computer aus Steuerdaten für eine Bearbeitungsmaschine zur Formung eines ophthalmologischen Implantats und/oder Kontrolldaten der Formung eines ophthalmologischen Implantats, insbesondere mittelseines spanabhebenden Drehverfahrens oder eines Laserstrahlformungsverfahrens, oder aus Steuerdaten für eine Bearbeitungsmaschine zur Formung und/oder Kontrolldaten der Formung einer Urform für die Herstellung eines ophthalmologischen Implantats Daten, insbesondere Steuerdaten für die Bearbeitungsmaschine, zur Durchführung der strukturellen Codierung von Identifikationsdaten des ophthalmologischen Implantats oder zur Ablage der strukturellen Codierung in der Urform erzeugt.

Ein solches ophthalmologisches Implantat, für das mit Hilfe des Computerprogrammprodukts Daten für eine strukturelle Codierung erzeugt werden können, ist insbesondere eine Intraokularlinse.

Der Computer, auf dem das Computerprogrammprodukt ausgeführt werden kann, kann dabei auch Teil einer Steuereinheit einer Drehmaschine oder eines Lasersystems sein.

Der Computer, auf dem das Computerprogrammprodukt ausgeführt werden kann, kann alternativ auch Teil einer Steuereinheit einer Bearbeitungsmaschine zur Herstellung einer Urform sein.

Insbesondere kann das Computerprogrammprodukt beispielsweise eingerichtet sein, aus der numerischen Linsentypbezeichnung und dem Brechwert einer gerade mittels eines spanabhebenden Drehverfahrens oder eines Laserstrahlformungsverfahrens in Herstellung befindlichen Intraokularlinse eine Modulationsstruktur für die dem Linsentyp und der Brechkraft entsprechende strukturellen Codierung zu errechnen und vorzugsweise gleich in Steuerdaten zu deren Erzeugung umzusetzen. Mit diesem Steuerdaten kann z. B. die Furchentiefe, Furchenbreite oder Neigungswinkel der Furche in der CNC-Datei eines Drehverfahrens gesteuert werden.

Bei Nutzung einer Urform zur Herstellung eines ophthalmologischen Implantats kann das Computerprogrammprodukt hingegen insbesondere eingerichtet sein, aus der numerischen Linsentypbezeichnung und dem Brechwert einer gerade in Herstellung befindlichen Urform für eine Intraokularlinse eine Modulationsstruktur für die dem Linsentyp und der Brechkraft entsprechende rotationssymmetrische strukturelle Codierung zu errechnen und vorzugsweise gleich in Steuerdaten zu deren Erzeugung umzusetzen. Mit diesen Steuerdaten kann auch hier z. B. die Furchentiefe, Furchenbreite oder Neigungswinkel der Furche in der CNC-Datei gesteuert werden, wenn die Urform beispielsweise mittels eines Drehverfahrens hergestellt wird.

Auf einem erfindungsgemäßen computerlesbaren Medium ist das oben beschrieben Computerprogrammprodukt gespeichert.

Die vorliegende Erfindung soll nun anhand von Ausführungsbeispielen erläutert werden. Es zeigt:
- die Fig. 1a ein erstes Ausführungsbespiel eines erfindungsgemäßen ophthalmologischen Implantats mit struktureller Codierung, die Fig. 1b das entsprechende Zustandsdiagramm von dessen struktureller Codierung, die Fig. 1c eine prinzipielle Ansicht dieser strukturellen Codierung von oben und die Fig. 1d einen Schnitt durch die strukturelle Codierung dieses Implantats.
- die Fig. 2a ein zweites Ausführungsbeispiel eines erfindungsgemäßen ophthalmologischen Implantats mit struktureller Codierung, die Fig. 2b das entsprechende Zustandsdiagramm von dessen struktureller Codierung und die Fig. 2c eine prinzipielle Ansicht dieser strukturellen Codierung von oben.
- die Fig. 3 ein drittes Ausführungsbeispiel eines erfindungsgemäßen ophthalmologischen Implantats mit struktureller Codierung.
- die Fig. 4 ein erfindungsgemäßes Charakterisierungssystem, das Teil eines entsprechenden Mikroskopiesystems ist.

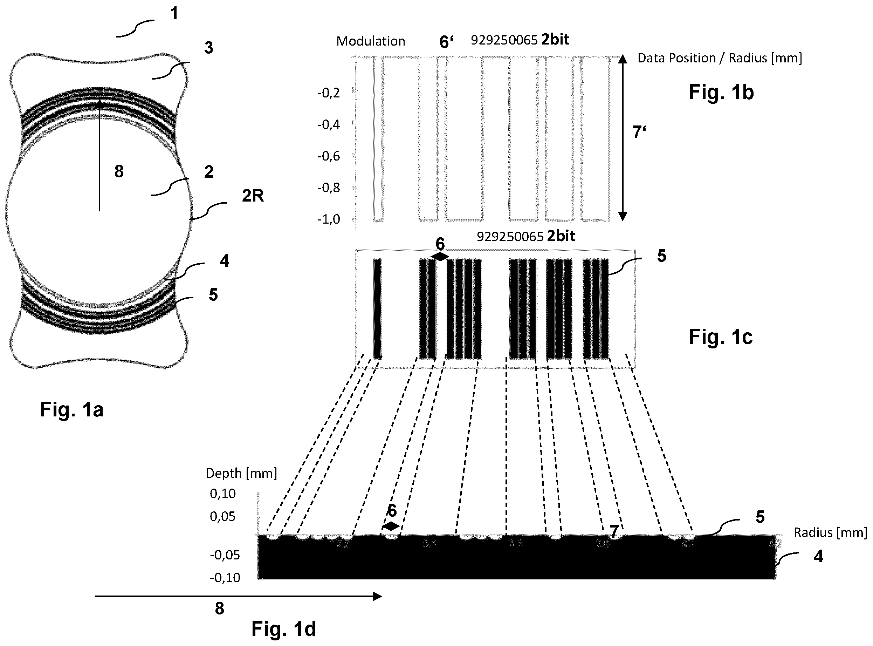

In der Fig. 1a ist ein erstes Ausführungsbeispiel eines erfindungsgemäßen ophthalmologischen Implantats 1 mit struktureller Codierung 5 dargestellt. Bei diesem ophthalmologischen Implantat 1 handelt es sich um eine Intraokularlinse, umfassend eine zentrale optische Linse 2, die das optisch abbildende Element dieses ophthalmologischen Implantats 1 darstellt, und eine Haptik 3 mit einer Haptikwurzel 4, die sich der zentralen optischen Linse 2 entlang eines Teilbereichs des umlaufenden Rands 2R dieser zentralen optischen Linse 2 anschließt, und diesen dabei von zwei Seiten teilweise umschließt. Auf der der Haptikwurzel 4 und dem der Haptikwurzel 4 nahen Bereich der Haptik 3 dieser Intraokularlinse ist eine strukturelle Codierung 5 von Identifikationsdaten des ophthalmologischen Implantats 1 angeordnet. Hierbei handelt es sich in diesem Fall um die strukturelle Codierung 5 des Typs und der Brechkraft dieser Intraokularlinse. In diesem Fall wurde eine strukturelle Codierung 5 in Form eines Barcodesystems genutzt, dass rotationssymmetrisch um die zentrale optische Linse 2 herum auf der Haptikwurzel 4 und auf der Haptik 3 in nächster Nähe zur zentralen optischen Linse 2 angeordnet ist. Die hier durchgeführte strukturelle Codierung 5 mittels Furchen, die mit demselben Diamantdrehverfahren in derselben Diamantdrehmaschine direkt in bzw. nach dem Formungsprozess der Intraokularlinse erzeugt wurde, nutzt eine 2-Bit-Lösung: Mit jeweils fester Furchenbreite 6 werden an unterschiedlichen radialen Positionen Furchen erzeugt (Furchentiefe 7 "1") bzw. keine Furchen erzeugt (Furchentiefe 7 "0").

Es wurde sich also in diesem Fall auf wenige Modulationsparameter beschränkt: Die radiale Position der Furche bei jeweils gleicher Furchenbreite 6, an der jeweils der Zustand 1 oder 0 möglich ist. Eine solche binäre strukturelle Codierung 5 ist deshalb mit ganz einfachen Mitteln erzeugbar und auch wieder auslesbar, denn es muss ja nur festgestellt werden, ob an der jeweiligen radialen Position 8 eine Furche vorliegt oder nicht.

Die Fig. 1b zeigt dann das entsprechende Zustandsdiagramm der strukturellen Codierung 5 dieses ersten Ausführungsbeispiels des erfindungsgemäßen ophthalmologischen Implantats 1, also der ersten Intraokularlinse, in der die oben beschriebenen zwei Zustände möglich sind. Die Fig. 1c zeigt eine prinzipielle Ansicht dieser strukturellen Codierung 5 von oben und die Fig. 1d einen Schnitt durch die strukturelle Codierung 5 dieses Implantats 1. Durch gestrichelte Linien wurden gleiche Bereiche einander zugeordnet.

Konkret ist in diesem ersten Ausführungsbeispiel eine 2-Bit-Codierung dargestellt, welche sich über eine räumliche Breite von ungefähr 1.0 mm erstreckt. Eine reine Tiefenmodulation der Furchentiefe 7 zur Erzeugung der beiden Zustände 0 und 1 wäre beispielsweise mit einer Amplitude von 5 µm - 15 µm bei einem Werkzeugradius von 10µm realisierbar.

Wird die in diesem ersten Ausführungsbeispiel mit 2-Bit-Codierung erzeugte strukturelle Codierung 5 dann decodiert, so zeigen sich die Identifikationsdaten zur Kennzeichnung dieser Intraokularlinse: Es handelt sich um eine AT LARA toric 929 mit einer Brechkraft von SE = 25.0D und einer Zylinderkorrektur von CYL = 6.5D.

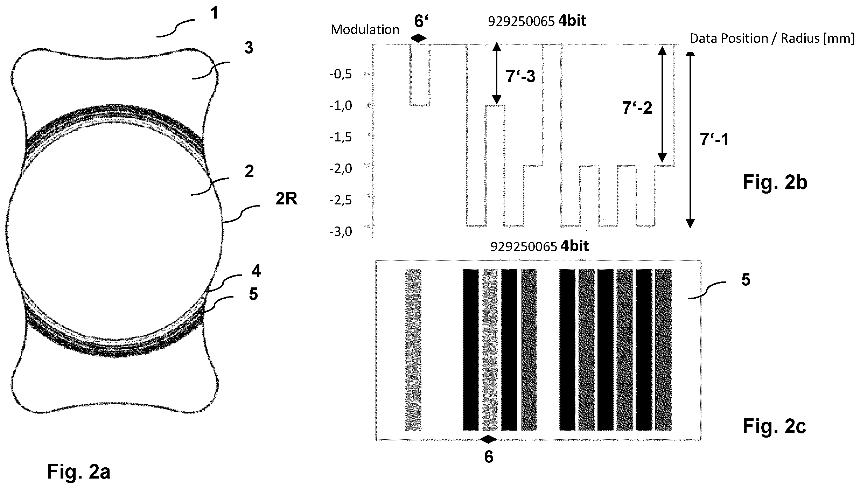

In der Fig. 2a ist ein zweites Ausführungsbeispiel eines erfindungsgemäßen ophthalmologischen Implantats 1 mit struktureller Codierung 5, dargestellt. Es handelt sich wiederum um eine Intraokularlinse, nämlich desselben Typs und derselben Brechkraft, allerdings mit einer strukturellen Codierung 5, die weitere Modulationsparameter enthält und somit eine räumliche Kompression der Datenstruktur erlaubt: Hier erfolgt eine zusätzliche Modulation der Furchentiefe 7 in mehreren Stufen 7'-1, 7'-2, 7'-3. Wie aus diesem zweiten Ausführungsbeispiel ersichtlich, benötigt der gleiche Dateninhalt mit einer solchen 4-Bit-Codierung nur noch ungefähr die halbe räumliche Breite.

Zu deren Decodierung wird dann allerdings eine höhere Auflösung eines entsprechenden Charakterisierungssystems 10 zur Identifikation dieser Intraokularlinse benötigt als zur Decodierung des ersten Ausführungsbeispiels: Eine Tiefenauflösung der Furchentiefe, derart, dass die verschiedenen Stufen 7'-1, 7'-2, 7'-3 voneinander unterschieden werden können, ist hier erforderlich.

Wird die in diesem zweiten Ausführungsbeispiel mit 4-Bit-Codierung erzeugte strukturelle Codierung 5 dann decodiert, so zeigen sich auch hier die Identifikationsdaten zur Kennzeichnung dieser Intraokularlinse. Es handelt sich auch hier um eine AT LARA toric 929 mit einer Brechkraft von SE = 25.0D und einer Zylinderkorrektur von CYL = 6.5D.

Die Modulationsparameter können, wie oben schon beschrieben, neben der schon beschriebenen radialen Position 8 der Furchen und der Furchentiefe 7 beispielsweise auch noch die Furchenbreite 6 und/oder den Neigungswinkel der Furche umfassen, um die strukturelle Codierung 5 weiter zu komprimieren und dieselben Identifikationsdaten auch noch geringerer räumlicher Breite unterzubringen, oder aber auf gleicher räumlicher Breite wesentlich mehr Informationen, also zusätzliche Identifikationsdaten, unterzubringen. Was hier möglich ist, als Modulationsparameter einzusetzen hängt davon ab, mit welchem optischen Detektionsverfahren und welchem Charakterisierungssystem 10 gearbeitet werden kann und ob folglich der entsprechende Modulationsparameter damit erfasst werden kann.

Die Fig. 2b zeigt dann das entsprechende Zustandsdiagramm der strukturellen Codierung 5 dieses zweiten Ausführungsbeispiels des erfindungsgemäßen ophthalmologischen Implantats 1, also der zweiten Intraokularlinse, in der die oben beschriebenen vier Zustände (vier verschiedenen Tiefen möglich sind. Die Fig. 2c zeigt eine prinzipielle Ansicht dieser strukturellen Codierung 5 von oben.

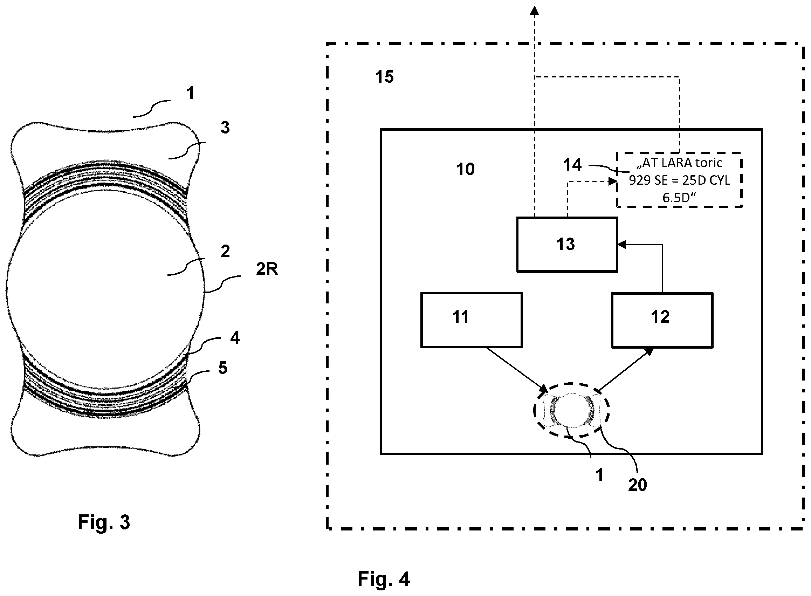

In der Fig. 3 ist ein drittes erfindungsgemäßes ophthalmologisches Implantat 1 mit struktureller Codierung 5 dargestellt, bei dem es sich um eine Intraokularlinse des Tys AT LISA tri 809 mit einer Brechkraft von 25.0D handelt
In der Fig. 4 ist schließlich ein erfindungsgemäßes Charakterisierungssystem 10 zur Identifikation eines ophthalmologischen Implantats 1, insbesondere einer Intraokularlinse dargestellt, das Teil eines entsprechenden Mikroskopiesystems 15 ist.

Dieses Ausführungsbeispiel eines erfindungsgemäßen Charakterisierungssystems 10 weist ein Beleuchtungssystem 11 zur Beleuchtung einer strukturellen Codierung 5 einer Intraokularlinse, ein Kamerasystem 12 zur Aufnahme von mittels der Beleuchtung detektierbar gemachten Strukturen der strukturellen Codierung 5 der Intraokularlinse, eine Analyseeinheit 13 zur Auswertung eines vom Kamerasystem 12 aufgenommenen Bildes der mittels der Beleuchtung detektierbar gemachten Strukturen der strukturellen Codierung 5 sowie zur Decodierung von Identifikationsdaten zur Identifikation des ophthalmologischen Implantats 1 aus diesem Bild sowie eine Anzeige- und/oder Ausgabevorrichtung 14 zur Anzeige und/oder Ausgabe der decodierten Identifikationsdaten des ophthalmologischen Implantats 1 auf.

Dieses Ausführungsbeispiel des erfindungsgemäßen Charakterisierungssystems 10 kann dabei auch Intraokularlinsen decodieren, die bereits in ein Patientenauge 20 implantiert sind.

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar, ohne den Rahmen der vorliegenden Erfindung zu verlassen.

Eine auf Verfahrensmerkmale bezogene Beschreibung einer Vorrichtung gilt bezüglich dieser Merkmale analog für das entsprechende Verfahren, während Verfahrensmerkmale entsprechend funktionelle Merkmale der beschriebenen Vorrichtung darstellen.

## Patentansprüche

1. Ophthalmologisches Implantat (1), insbesondere Intraokularlinse, umfassend ein optisch abbildendes Element (2), insbesondere eine zentrale optische Linse, und eine Haptik (3) mit einer Haptikwurzel (4), die sich dem optisch abbildenden Element (3) anschließt, dieses an einem umlaufenden Rand (2R) mindestens teilweise umschließt, **dadurch gekennzeichnet, dass** auf der Haptikwurzel (4) und/oder dem der Haptikwurzel (4) nahen Bereich der Haptik (3) eine rotationssymmetrische strukturelle Codierung (5) von Identifikationsdaten des ophthalmologischen Implantats (1), insbesondere des Typs und der Brechkraft, mittels eines Barcodesystems in Form eines ringförmigen Barcodes, der im gesamten Bereich der Haptikwurzel und/oder der der Haptikwurzel nahen Bereich der Haptik um den umlaufenden Rand des optisch abbildenden Elements herum angeordnet ist.

2. Ophthalmologisches Implantat (1) nach Anspruch 1, dessen rotationssymmetrische strukturelle, also eine Topographie aufweisende, Codierung (5) mittels eines Drehverfahrens, insbesondere eines Diamantdrehverfahrens, oder eines Laserstrahlformungsverfahrens direkt während oder nach der Formung des ophthalmologischen Implantats (1) erzeugt ist.

3. Ophthalmologisches Implantat (1) nach Anspruch 1 oder 2, **gekennzeichnet durch** eine Codierung, die durch ein Set räumlicher Modulationsparameter bestimmt ist.

4. Ophthalmologisches Implantat (1) nach Anspruch 3, **gekennzeichnet durch** eine Codierung, die durch mindestens einen der folgenden Modulationsparameter beschrieben ist: Furchenbreite (6), Furchentiefe (7), Neigungswinkel der Furche, Position (8) der Furche, insbesondere eine radiale Position (8) der Furche.

5. Ophthalmologisches Implantat (1) nach einem der Ansprüche 1 bis 4,
wobei das optisch abbildende Element (2) und die Haptik (3) mit ihrer Haptikwurzel (4) aus einem Werkstück gefertigt sind.

6. Verfahren zur Herstellung eines ophthalmologischen Implantats (1), insbesondere einer Intraokularlinse, nach einem der Ansprüche 1 bis 5, in dem das ophthalmologische Implantat (1) mittels eines spanabhebenden Drehverfahrens, insbesondere mittels eines Diamantdrehverfahrens, oder mittels eines Laserstrahlformungsverfahrens geformt wird und direkt während oder nach der Formung des ophthalmologischen Implantats (1) eine rotationssymmetrische strukturelle Codierung (5) von Identifikationsdaten des ophthalmologischen Implantats (1), insbesondere des Typs und der Brechkraft, mittels des spanabhebenden Drehverfahrens oder des Laserstrahlformungsverfahrens erfolgt.

7. Verfahren nach Anspruch 6, in dem Daten, insbesondere Steuerdaten, zur Durchführung der strukturellen Codierung von Identifikationsdaten des ophthalmologischen Implantats (1) aus Steuerdaten zur Formung des ophthalmologischen Implantats (1) und/oder aus Kontrolldaten der Formung des ophthalmologischen Implantats (1) gewonnen werden.

8. Computerprogrammprodukt mit Programm-Code, der bei seiner Ausführung auf einem Computer aus Steuerdaten für eine Bearbeitungsmaschine zur Formung eines ophthalmologischen Implantats (1) und/oder Kontrolldaten der Formung eines ophthalmologischen Implantats (1) gemäß einem der Ansprüche 1 bis 5 oder aus Steuerdaten für eine Bearbeitungsmaschine zur Formung und/oder Kontrolldaten der Formung einer Urform für die Herstellung eines ophthalmologischen Implantats (1) gemäß einem der Ansprüche 1 bis 5 Daten, insbesondere Steuerdaten für die Bearbeitungsmaschine, zur Durchführung der strukturellen Codierung von Identifikationsdaten des ophthalmologischen Implantats (1) oder zur Ablage der strukturellen Codierung in der Urform erzeugt.

## Claims

1. Ophthalmological implant (1), in particular an intraocular lens, comprising an optically imaging element (2), in particular a central optical lens, and a haptic (3) with a haptic root (4) which adjoins the optically imaging element (2) and at least partly encloses the latter at a circumferential edge (2R), **characterized in that** a rotationally symmetric structural coding (5) of identification data of the ophthalmological implant (1), in particular relating to the type and the refractive power, is arranged on the haptic root (4) and/or the region of the haptic (3) close to the haptic root (4) by means of a barcode system in the form of a ring-shaped barcode, which is arranged around the circumferential edge of the optically imaging element in the entire region of the haptic root and/or the region of the haptic close to the haptic root.

2. Ophthalmological implant (1) according to Claim 1, wherein the rotationally symmetric structural coding (5) thereof, said structural coding having a topography, is generated by means of a lathing and/or milling method, in particular a diamond lathing and/or milling method, or a laser beam forming method directly during or after the forming of the ophthalmological implant (1).

3. Ophthalmological implant (1) according to Claim 1 or 2, **characterized by** a code which is determined by a set of spatial modulation parameters.

4. Ophthalmological implant (1) according to Claim 3 **characterized by** a code which is described by at least one of the following modulation parameters: groove width (6), groove depth (7), inclination angle of the groove, position (8) of the groove, in particular a radial position (8) of the groove.

5. Ophthalmological implant (1) according to any of Claims 1 to 4,
wherein the optically imaging element (2) and the haptic (3) with its haptic root (4) are manufactured from one workpiece.

6. Method for producing an ophthalmological implant (1), in particular an intraocular lens, according to any of Claims 1 to 5, wherein the ophthalmological implant (1) is formed by means of a machining lathing and/or milling method, in particular by means of a diamond lathing and/or milling method, or by means of a laser beam forming method and rotationally symmetric structural coding (5) of identification data of the ophthalmological implant (1), in particular of the type and of the refractive power, is implemented by means of the machining lathing and/or milling method or the laser beam forming method directly during or after the forming of the ophthalmological implant (1).

7. Method according to Claim 6, wherein data, in particular control data, for carrying out the structural coding of identification data of the ophthalmological implant (1) are obtained from control data for forming the ophthalmological implant (1) and/or from monitoring data of the forming of the ophthalmological implant (1).

8. Computer program product with program code which, upon execution on a computer, generates data, in particular control data for a processing machine, to carry out the structural coding of identification data of an ophthalmological implant (1) or to deposit the structural coding in a primary mould, said data being generated from control data for a processing machine for forming the ophthalmological implant (1) and/or from monitoring data of the forming of an ophthalmological implant (1) according to any of Claims 1 to 5 or from control data for a processing machine for forming a primary mould and/or from monitoring data of the forming of said primary mould for the production of an ophthalmological implant (1) according to any of Claims 1 to 5.

## Revendications

1. Implant ophtalmologique (1), en particulier lentille intraoculaire, comprenant un élément d'imagerie optique (2), en particulier une lentille optique centrale, et un élément haptique (3) avec une racine haptique (4) qui se raccorde à l'élément d'imagerie optique (2), qui l'entoure au moins en partie sur un bord périphérique (2R), **caractérisé en ce qu'**est disposé sur la racine haptique (4) et/ou la zone de l'élément haptique (3) proche de la racine haptique (4) un codage structurel (5) à symétrie de rotation de données d'identification de l'implant ophtalmologique (1), en particulier du type et de la force réfractive, au moyen d'un système de code à barres sous forme d'un code à barres annulaire, qui est disposé tout autour du bord périphérique de l'élément d'imagerie optique dans la totalité de la zone de la racine haptique et/ou dans la zone de l'élément haptique proche de la racine haptique.

2. Implant ophtalmologique (1) selon la revendication 1, dont le codage structurel (5) à symétrie de rotation, comportant donc une topographie, est généré directement pendant ou après le façonnage de l'implant ophtalmologique (1) au moyen d'un procédé de meulage, en particulier d'un procédé de meulage au diamant, ou d'un procédé de façonnage par faisceau laser.

3. Implant ophtalmologique (1) selon la revendication 1 ou 2, **caractérisé par** un codage, qui est déterminé par un ensemble de paramètres de modulation spatiale.

4. Implant ophtalmologique (1) selon la revendication 3, **caractérisé par** un codage, qui est décrit par au moins un des paramètres de modulation suivants : largeur (6) de sillon, profondeur (7) de sillon, angle d'inclinaison du sillon, position (8) du sillon, en particulier position radiale (8) du sillon.

5. Implant ophtalmologique (1) selon l'une des revendications 1 à 4, **caractérisé en ce que** l'élément d'imagerie optique (2) et l'élément haptique (3) avec sa racine haptique (4) sont produits à partir d'une pièce.

6. Procédé de fabrication d'un implant ophtalmologique (1), en particulier d'une lentille intraoculaire, selon l'une des revendications 1 à 5, dans lequel l'implant ophtalmologique (1) est façonné au moyen d'un procédé de meulage par enlèvement de copeaux, en particulier au moyen d'un procédé de meulage au diamant, ou au moyen d'un procédé de façonnage par faisceau laser et, directement pendant ou après le façonnage de l'implant ophtalmologique (1), un codage structurel (5) à symétrie de rotation de données d'identification de l'implant ophtalmologique (1), en particulier du type et de la force de réfraction, est effectué au moyen du procédé de meulage par enlèvement de copeaux ou du procédé de façonnage par faisceau laser.

7. Procédé selon la revendication 6, dans lequel des données sont obtenues, en particulier des données de commande, pour mettre en œuvre le codage structurel de données d'identification de l'implant ophtalmologique (1) à partir de données de commande pour le façonnage de l'implant ophtalmologique (1) et/ou à partir de données de contrôle du façonnage de l'implant ophtalmologique (1).

8. Produit-programme d'ordinateur avec un code de programme, qui génère, lors de son exécution sur un ordinateur, à partir de données de commande pour une machine d'usinage pour façonner un implant ophtalmologique (1) et/ou de données de contrôle du façonnage d'un implant ophtalmologique (1) selon l'une des revendications 1 à 5 ou de données de commande pour une machine d'usinage pour le façonnage et/ou de données de contrôle du façonnage d'une forme d'origine pour la fabrication d'un implant ophtalmologique (1) selon l'une des revendications 1 à 5, des données, en particulier des données de commande pour la machine d'usinage, pour mettre en œuvre le codage structurel de données d'identification de l'implant ophtalmologique (1) ou pour stocker le codage structurel sous sa forme d'origine.
